# EUROPEAN PATENT APPLICATION

(11) **EP 1 992 714 A1**
(43) Date of publication of application: **19.11.2008**
(21) Application number: 08156411.4
(22) Date of filing: 16.05.2008
(51) Int. Cl.: C23C 14/00, C07F 3/00

(54) **Group 2 Metal Precursors for Depositing Multi-Component Metal Oxide Films**

(30) Priority: 16.05.2007 US 938233 P; 01.05.2008 US 113397
(71) Applicant: Air Products and Chemicals, Inc., Allentown, PA 18195-1501 (US)
(72) Inventor: Lei, Xinjian, Vista,, CA 92081 (US); Quinn, Liam J., 10088, Taipei, Sec. 3 (TW); Spence, Daniel P., San Diego,, CA 92117 (US)
(74) Representative: Muir, Benjamin M. J.

(57) **Abstract**

Novel Sr and Ba complexes containing both beta-diketonates and N-methyl-pyrrolidone were synthesized and characterized. TGA experiments indicated these complexes are volatile, they can be employed as potential precursors for ALD strontium titanate (STO) or barium strontium titanate films (BST) films in semiconductor fabrication.

## Description

### BACKGROUND OF THE INVENTION

The semiconductor fabrication industry has an interest in depositing barium strontium titanate films ("BST") for constructing various electronic devices in integrated circuits. The industry has sought precursors of barium strontium and titanium that are stable, liquid and readily decompose under standard deposition conditions leaving high purity BST films and removing in the vapor phase the remainder of the precursors of barium, strontium and titanium, typically in the form of ligands that reversibly bind the metals until the deposition conditions are provided wherein the metal is deposited from the ligand precursor resulting in essentially deposited metal and volatile, gas phase leaving groups constituting the ligand that bound the metal in the precursor form or decomposition components of the ligand which still exhibit the properties of being good leaving groups that do not contaminate the deposited BST film and typically leave as volatile, gaseous groups.

Representative art to this field includes:
Auld, J.; Jones, A. C.; Leese, A. B.; Cockayne, B.; Wright, P. J.; O'Brien, P.; Motevalli, M., "Vapor-phase transport of barium b-diketonates in the deposition of oxides and the isolation and structural characterization of a novel hexameric cluster of 2,2,6,6-tetramethylheptane-3,5-dionato-barium(II)", Journal of Materials Chemistry, 3, (12), 1203-8, (1993).
Baum, T. H., "Alkane and polyamine solvent compositions for liquid delivery chemical vapor deposition", 99-454954 6344079, 19991203., (2002).
Baum, T. H.; Paw, W., "Group IIA β-diketonate tetrahydrofuran-adduct complexes as source reagents for chemical vapor deposition", 99-321637 6218518,19990528.,(2001).
Bedoya, C.; Condorelli, G. G.; Motta, A.; Mauro, A. D.; Anastasi, G.; Fragalà, I. L.; Lisoni, J. G.; Wouters, D., "MOCVD of Sr-Containing Oxides: Transport Properties and Deposition Mechanisms of the Sr(tmhd)2·pmdeta Precursor", Chemical Vapor Deposition, 11, 269 - 275, (2005).
Brooks, J.; Davies, H. O.; Leedham, T. J.; Jones, A. C.; Steiner, A., "The crystal structure of unadducted strontium bis- tetramethylheptanedionate: The standard precursor for the MOCVD of strontium-containing oxides", Chemical Vapor Deposition, 6, (2), 66+, (2000).
Cheol Seong, H.; Jaehoo, P.; Doo Sup, H.; Cha Young, Y., Compositional Variation of Metallorganic Chemically Vapor Deposited SrTiO3 Thin Films along the Capacitor Hole Having a Diameter of 0.15 mu m. In ECS: (2001); Vol. 148, pp G636-G639.
Drake, S. R.; Miller, S. A. S.; Williams, D. J., "Monomeric Group IIA metal β - diketonates stabilized by multidentate glymes", Inorganic Chemistry, 32, (15), 3227-35, (1993).
Drozdov, A. A.; Trojanov, S. I., "New oligomeric structures of barium dipivaloylmethanate, Ba4(tmhd)8, and its pivalate derivative Ba5(tmhd)9(piv)", Polyhedron 11, 2877-2882 (1992).
Gardiner, R. A.; Gordon, D. C.; Stauf, G. T.; Vaartstra, B. A., "Mononuclear Barium Diketonate Polyamine Adducts - Synthesis, Structures, and Use in MOCVD of Barium-Titanate", Chemistry of Materials, 6, (11), 1967-1970, (1994).
Ghandari, G.; Glassman, T. E.; Studebaker, D. B.; Stauf, G.; Baum, T. H., "Comparison of (L)M(tmhd)2(M = Mg, Ca, Sr, Ba; L = tetraglyme, pmdeta) precursors for high K dielectric MOCVD", Materials Research Society Symposium Proceedings, 446, (Amorphous and Crystalline Insulating Thin Films--1996), 327-332, (1997).
Glassman, T. E.; Bhandari, G.; Baum, T. H., "Evidence for cooperative oxidation of MOCVD precursors used in BaxSr1-xTiO3 film growth", Materials Research Society Symposium Proceedings, 446, (Amorphous and Crystalline Insulating Thin Films--1996), 321-326, (1997).
Hintermaier, F. S.; Baum, T. H., "Alkane and polyamine solvent compositions for liquid delivery chemical vapor deposition", 98-185374 6214105, 19981103., (2001).
Hubertpfalzgraf, L. G.; Labrize, F., "Barium Beta-Diketonate Derivatives with Aminoalcohols - Synthesis, Molecular-Structure and Thermal-Behavior of Ba5(µ4-OH)(µ3,η2-OCH(Me)CH2NMe2)4(µ, η 2- tmhd)4(η2-tmhd) and of [Ba(µ,η2:η2-tmhd)(η2-tmhd)(η2-OHCH(Me)CH2NMe2)]2", Polyhedron, 13, (14), 2163-2172, (1994).
Hwang, C. S.; No, S. Y.; Park, J.; Kim, H. J.; Cho, H. J.; Han, Y. K.; Oh, K. Y., "Cation Composition Control of MOCVD (Ba,Sr)TiO3 Thin Films along the Capacitor Hole", Journal of The Electrochemical Society, 149, (10), G585-G592, (2002).
Kwon, O. S.; Kim, S. K.; Cho, M.; Hwang, C. S.; Jeong, J., "Chemically Conformal ALD of SrTiO3 Thin Films Using Conventional Metallorganic Precursors", Journal of The Electrochemical Society, 152, (4), C229-C236, (2005).
Kwon, O. S.; Lee, S. W.; Han, J. H.; Hwang, C. S., "Atomic Layer Deposition and Electrical Properties of SrTiO3 Thin Films Grown Using Sr(C11H19O2)2, Ti(Oi-C3H7)4, and H2O", J. Electrochem. Soc., 154, G127-G133, (2007).
Min, Y.-S.; Cho, Y. J.; Kim, D.; Lee, J.-H.; Kim, B. M.; Lim, S. K.; Lee, I. M.; Lee, W. I., "Liquid source-MOCVD of BaxSr1-xTiO3 (BST) thin films with a N-alkoxy-b-ketoiminato titanium complex", Chemical Vapor Deposition, 7, (4), 146-149, (2001).
Ohkawa, A.; Tsutsumi, Y., "Deposition of thin films in fabrication of semiconductor devices by MOCVD process", 98-263702 2000100802, 19980917., (2000).
Paw, W.; Baum, T. H., "Preparation of Group IIA metal b-diketonate Lewis base MOCVD source reagents, and method of forming Group IIA metal-containing films utilizing same", 6338873, Jul 6, 2000, (2002).
Roeder, J. F.; Stauf, G. T., "Liquid delivery MOCVD process for deposition of high frequency dielectric materials", 99-US29566 2000037712, 19991213., (2000).
Rossetto, G.; Polo, A.; Benetollo, F.; Porchia, M.; Zanella, P., "Studies on Molecular Barium Precursors for Mocvd - Synthesis and Characterization of Barium 2,2,6,6-Tetramethyl-3,5- Heptanedionate - X-Ray Crystal-Structure of [Ba(tmhd)2.Et2O]2", Polyhedron, 11, (8), 979-985, (1992).
Stauf, G. T.; Roeder, J. F.; Baum, T. H., "Liquid delivery MOCVD process for deposition of high frequency dielectric materials", 98-216673 6277436, 19981218., (2001).

### BRIEF SUMMARY OF THE INVENTION

The present invention is in a first aspect directed to a metal containing complex represented by a structure selected from: 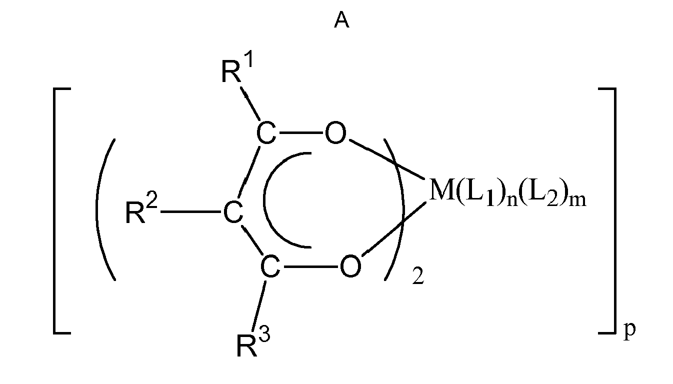 wherein M is selected from Mg, Ca, Sr, and Ba; R¹ and R³ are independently selected from linear or branched alkyl, alkoxyalkyl, fluoroalkyl: having from 1 to 10 carbon atoms, cycloaliphatic, and aryl having from 4 to 12 carbon atoms; R² is independently selected from hydrogen, linear or branched alkyl, fluoroalkyl, alkoxy: having from 1 to 10 carbon atoms, cycloaliphatic, and aryl: having from 4 to 12 carbon atoms; L₁ is independently selected from the organic amide class RCONR'R" wherein R and R' are independently linear or branched alkyl having from 1-10 carbon atoms and they can be connected to form a cyclic group (CH₂)_{q}, wherein q is from 4-6, and R" is selected from alkyl having from 1 to 4 carbon atoms and cycloalkyl having from 4 to 8 carbon atoms; L₂ is independently selected from H₂O, ROH wherein R is linear or branched alkyl having from 1-10 carbon atoms or a cyclic group (CH₂)_{q} wherein q is from 4-6, and an organic amide of the class RCONR'R" wherein R and R' are independently linear or branched alkyl having from 1-10 carbon atoms and they can be connected to form a cyclic group (CH₂)_{q} wherein q is from 4-6, and R" is selected from alkyl having from 1 to 4 carbon atoms and cycloalkyl having from 4 to 8 carbon atoms; n is a number selected from between 1 and 4; m is selected from a number between 0 to 4; and p is selected from 1, 2; 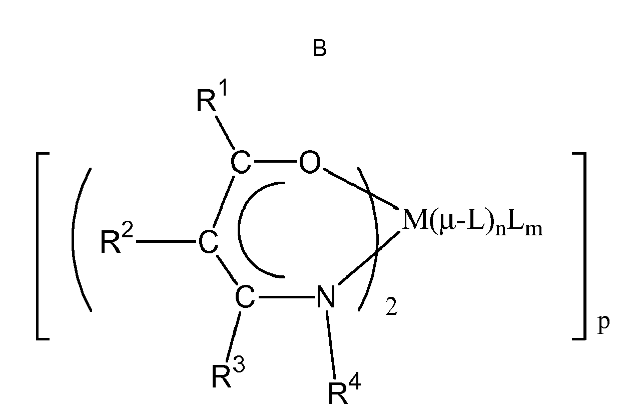 wherein M is selected from Mg, Ca, Sr, and Ba; R¹ and R⁴ are independently selected from linear or branched alkyl, alkoxyalkyl, fluoroalkyl: having from 1 to 10 carbon atoms, cycloaliphatic, and aryl: having from 4 to 12 carbon atoms; R² and R³ are independently selected from hydrogen, linear or branched alkyl, alkoxyalkyl, fluoroalkyl, alkoxy: having from 1 to 10 carbon atoms, cycloaliphatic, and aryl: having from 4 to 12 carbon atoms; L is independently selected from the organic amide class RCONR'R" wherein R and R' are independently linear or branched alkyl having from 1-10 carbon atoms and they can be connected to form a cyclic group (CH₂)_{q}, wherein q is from 4-6, and R" is selected from alkyl having from 1 to 4 carbon atoms and cycloalkyl having from 4 to 8 carbon atoms; n is a number selected from between 0 and 4 and m is selected from a number between 1 to 4, or n is a number selected from between 1 and 4 and m is a number selected from between 0 to 4; p is selected from 1, 2; and µ-L indicates L is connected to two metals, M, via µL's oxygen atom when p=2; and, 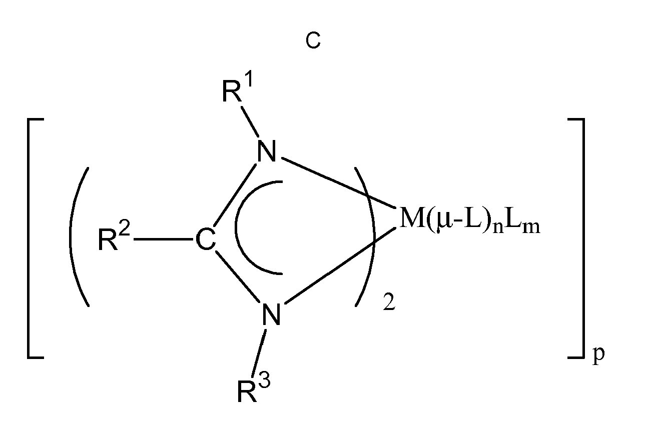 wherein M is selected from Mg, Ca, Sr, and Ba; R¹ and R³ are independently selected from linear or branched alkyl, alkoxyalkyl, fluoroalkyl: from 1 to 10 carbon atoms, cycloaliphatic, and aryl: having from 4 to 12 carbon atoms; R² is independently selected from hydrogen, linear or branched alkyl, fluoroalkyl, alkoxy: having from 1 to 10 carbon atoms, cycloaliphatic, and aryl: having from 4 to 12 carbon atoms; L is independently selected from the organic amide class RCONR'R" wherein R and R' are independently linear or branched alkyl having from 1-10 carbon atoms and they can be connected to form a cyclic group (CH₂)_{q}, wherein q is from 4-6, and R" is selected from alkyl having from 1 to 4 carbon atoms and cycloalkyl having from 4 to 8 carbon atoms; n is a number selected from between 0 and 4 and m is selected from a number between 1 to 4, or n is a number selected from between 1 and 4 and m is a number selected from between 0 to 4; p is selected from 1, 2; and µ-L indicates L is connected to two metals, M, via µL's oxygen atom when p=2.

In a preferred embodiment, n+m= 4 or less. Preferably, where p=2, n is a number selected from between 1 and 2, and m is a number selected from between 0 and 2.

M is preferably selected from calcium, strontium, barium and mixtures thereof.

L, L₁ and L₂ are preferably selected from organic amides such as N-methyl pyrrolidinone (NMP), *N,N*-Diethylformamide (DEF), *N,N*-Diethylacetamide (DEAC), *N,N-*Dimethylacetamide (DMAC), N-cyclohexyl 2-pyrrolidinone and, in the case of L₂, H₂O and ROH. In addition to NMP and N-cyclohexyl 2-pyrrolidinone, other N-substituted lactams (i.e. other organic amides of the class RCONR'R" wherein R and R' are alkyl and are connected to form with the C and N a 4-6 membered cyclic group, and R" is selected from alkyl having from 1 to 4 carbon atoms and cycloalkyl having from 4 to 8 carbon atoms) also constitute preferred organic amides.

Where the metal containing complex is of structure A and_p=2, L₁ is preferably connected to both metals, M, via L₁'s oxygen atom.

Where the metal containing complex is of structure A, R¹ and R³ are preferably selected from t-butyl and t-pentyl, and R² is preferably selected from hydrogen, methyl and ethyl.

In one embodiment, the metal containing complex is of structure A, M is strontium, R¹ and R³ are each t-butyl, R² is hydrogen, L₁ and L₂ are each N-methyl-pyrrolidone, n=1.5, m=0.5, and p=2.

In another embodiment, the metal containing complex is of structure A, M is strontium, R¹ and R³ are each t-butyl, R² is hydrogen, L₁ is *N,N*-Diethylacetamide (DEAC), n =1.5, m=0, and p=2.

In another embodiment, the metal containing complex is of structure A, M is barium, R¹ and R³ are each CF₃, R² is hydrogen, L₁ and L₂ are each N-methyl-pyrrolidone, n =1.5, m=2, and p=2.

In another embodiment, the metal containing complex is of structure A, M is barium, R¹ and R³ are each t-butyl, R² is hydrogen, L₁ is N N-methyl-pyrrolidone, n=2, L₂=H₂O, m=1, and p=2.

Where the metal containing complex is of structure B, R¹ and R³ are preferably independently selected from t-butyl and t-pentyl; R² is preferably selected from hydrogen, methyl and ethyl; and R⁴ is preferably selected from iso-propyl, t-butyl, sec-butyl, t-pentyl and mixtures thereof.

Where the metal containing complex is of structure C, R¹ and R³ are preferably independently selected from the t-butyl and t-pentyl; and R² is preferably selected from hydrogen, methyl and ethyl.

Exemplary preferred metal containing complexes include:
Ba₂(1,1,1,5,5,5-hexafluoro-2,4-pentanedione)₄(N-methyl pyrrolidinone)₅;
Sr₂(2,2,6,6-tetramethyl-3,5-heptanedione)₄(N-methyl pyrrolidinone)₄;
Sr₂(2,2,6,6-tetramethyl-3,5-heptanedione)₄(*N,N*-Diethylacetamide)₃;
Ba₂(2,2,6,6-tetramethyl-3,5-heptanedione)₄(N-methyl pyrrolidinone)₄·(H₂O)₂;
bis(2,2-dimethyl-5-(iso-propylamino)-3-hexanonato)barium(N-methyl pyrrolidinone);
bis(2,2-dimethyl-5-(sec-butylamino)-3-hexanonato)strontium(N-methyl pyrrolidinone); and
bis(2,2-dimethyl-5-(sec-butylamino)-3-hexanonato)strontium(N,N-Diethylacetamide).

In a preferred embodiment, the metal containing complex is dissolved in a solvent selected from glyme solvents having from 1 to 20 ethoxy -(C₂H₄O)- repeat units; C₂-C₁₂ alkanols; organic ethers; C₆-C₁₂ aliphatic hydrocarbons; C₆-C₁₈ aromatic hydrocarbons; organic esters; organic amines; and polyamines and organic amides. Organic ethers selected from dialkyl ethers comprising C₁-C₆ alkyl moieties, C₄-C₈ cyclic ethers, and C₁₂-C₆₀ crown O₄-O₂₀ ethers (wherein C is the number of carbon atoms in the crown ether compound and O is the number of oxygen atoms in the crown ether), and organic amides selected from N-methyl pyrrolidinone ("NMP"), *N,N-*Diethylformamide (DEF), *N,N-*Diethylacetamide (DEAC), *N,N-*Dimethylacetamide (DMAC),and N-cyclohexyl 2-pyrrolidinone, are in particular preferred.

According to a second aspect, the present invention is directed to the use of a metal containing complex of the first aspect to form a conformal metal oxide thin film on a substrate.

According to a third aspect, the present invention is directed to a vapor deposition process for forming a conformal multi-component metal oxide thin film on a substrate, wherein at least two metal containing complexes and an oxygen containing agent are introduced to a deposition chamber and a multi-component metal oxide film is deposited on a substrate, wherein the at least two metal containing complexes are each in accordance with the first aspect of the invention, and have different metals.

The vapor deposition process is preferably selected from chemical vapor deposition, cyclic chemical vapor deposition and atomic layer deposition.

The oxygen containing agent is preferably selected from N₂O, O₂, H₂O₂, H₂O, ozone, O₂ plasma, organic peroxide, and mixtures thereof.

The first metal containing complex is preferably selected from Sr₂(tmhd)₄(NMP)₄, and Sr₂(tmhd)₄(DEAC)₃, and the second metal containing complex is preferably selected from Ba₂(tmhd)₄(NMP)₄·(H₂O)₂, and Ba₂(hfac)₄(NMP)₅.

A third metal containing complex is preferably selected from titanium containing alkoxides and beta-ketonates and mixtures thereof. Preferably, the third metal containing complex is selected from Ti(OPrⁱ)₄, Ti(tmhd)₂(OPrⁱ)₂, where Prⁱ = isopropyl and tmhd = 2,2,6,6-tetramethyl-3,5-heptanedionate, Ti(mpd)(tmhd)₂, where mpd = 2-methyl-2,4-pentanedioxy, Ti(4-(2-methylethoxy)imino-2-pentanoate)₂, and mixtures thereof.

### BRIEF DESCRIPTION OF THE DRAWINGS

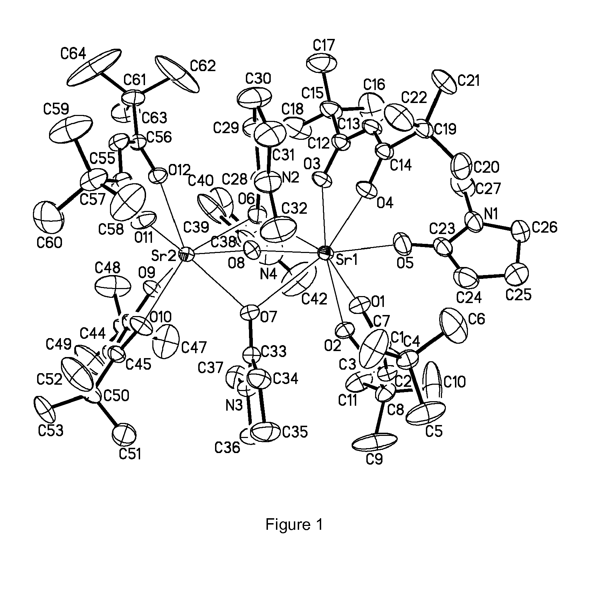

Figure 1 is a drawing representative of the crystal structure of Sr₂(tmhd)₄(NMP)₄.

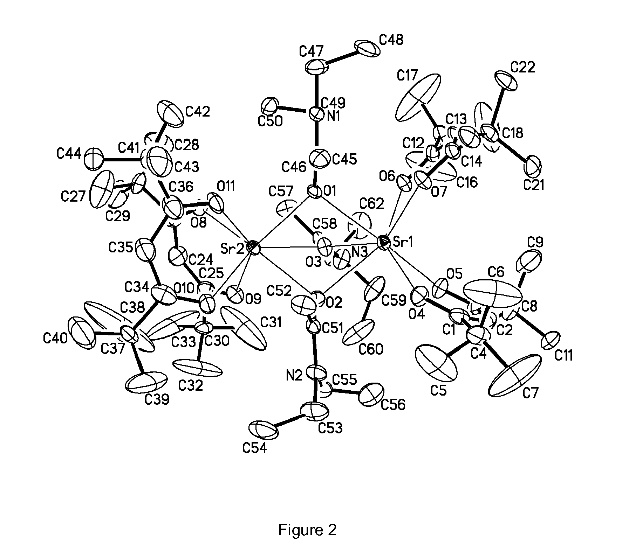

Figure 2 is a drawing representative of the crystal structure of Sr₂(tmhd)₄(DEAC)₃.

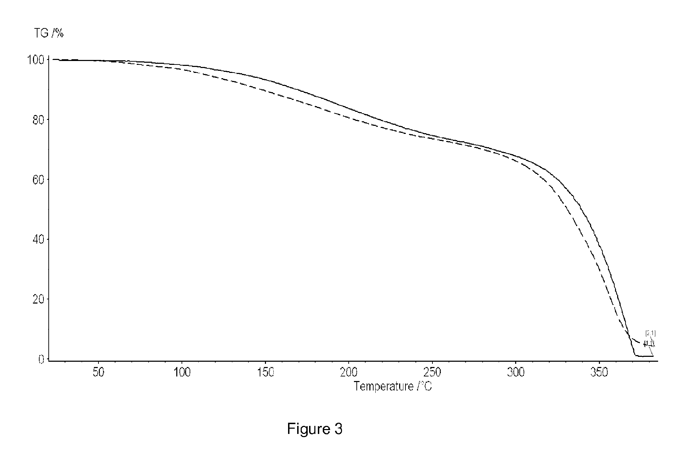

Figure 3 is TGAs of Sr₂(tmhd)₄(NMP)₄ (dashed line) and Sr₂(tmhd)₄(DEAC)₃ (solid line), showing both are volatile complexes.

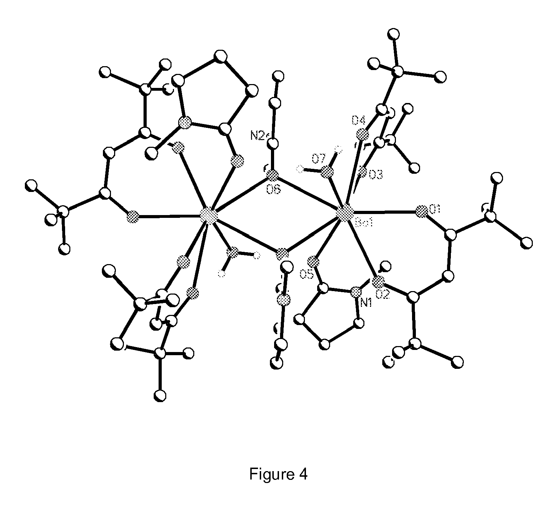

Figure 4 is a drawing representative of the crystal structure of [Ba(tmhd)₂(NMP)₂·H₂O]₂.

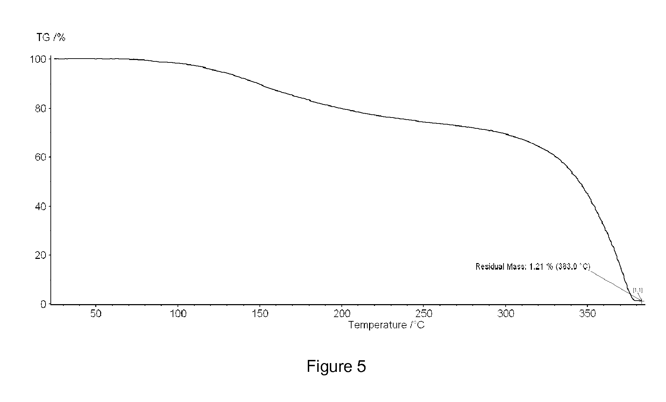

Figure 5 is TGA of [Ba(tmhd)₂(NMP)₂·H₂O]₂, indicating it is volatile.

### DETAILED DESCRIPTION OF THE INVENTION

Novel Sr and Ba complexes containing both beta-diketonates and N-methyl-pyrrolidone have been synthesized and characterized. Thermogravimetric Analysis (TGA) experiments indicate these complexes are volatile, and that they can be employed as potential precursors for chemical vapor deposition (CVD), cyclic chemical vapor deposition (CCVD), or atomic layer deposition (ALD) of strontium titanate (STO) or barium strontium titanate films (BST) films in semiconductor fabrication. In the deposition of STO and BST films, the titanium source is selected from titanium containing precursors exemplified by titanium alkoxides or beta-diketonates such as Ti(OPrⁱ)₄, Ti(tmhd)₂(OPrⁱ)₂, where Prⁱ = isopropyl, where tmhd = 2,2,6,6-tetramethyl-3,5-heptanedionate, Ti(mpd)(tmhd)₂, where mpd = 2-methyl-2,4-pentanedioxy, Ti(4-(2-methylethoxy)imino-2-pentanoate)₂, and analogous titanium ligands and derivatives.

These Group 2 metal complexes are precursors capable of depositing Group 2 metal-containing films for semiconductor applications. The metal complexes include:
(i) Group 2 beta-diketonate with organic amides as adducts with a formula of [M(R¹C(O)CR²C(O)R³)₂(L₁)ₙ(L₂)ₘ]ₚ wherein R¹ and R³ are independently selected from linear or branched alkyl, alkoxyalkyl, fluoroalkyl: having from 1 to 10 carbon atoms, cycloaliphatic, and aryl: having from 4 to 12 carbon atoms; R² is independently selected from hydrogen, linear or branched alkyl, fluoroalkyl, alkoxy: having from 1 to 10 carbon atoms, cycloaliphatic, and aryl: having from 4 to 12 carbon atoms; L₁ is independently selected from the organic amide class RCONR'R" wherein R and R' are independently linear or branched alkyl having from 1-10 carbon atoms and they can be connected to form a cyclic group (CH₂)_{q}, wherein q is from 4-6, and R" is selected from alkyl having from 1 to 4 carbon atoms and cycloalkyl having from 4 to 8 carbon atoms; L₂ is independently selected from the H₂O, ROH wherein R is linear or branched alkyl having from 1-10 carbon atoms or a cyclic group (CH₂)_{q} wherein q is from 4-6, and an organic amide of the class RCONR'R" wherein R and R' are independently linear or branched alkyl having from 1-10 carbon atoms and they can be connected to form a cyclic group (CH₂)_{q} wherein q is from 4-6, and R" is selected from alkyl having from 1 to 4 carbon atoms and cycloalkyl having from 4 to 8 carbon atoms; n is a number selected from between 1 and 4; m is selected from a number between 0 to 4; and p is selected from 1, 2; and,
(ii) Group 2 beta-ketoiminate with organic amides as adducts with a formula of [M(R¹C(O)CR²C(NR³)R⁴)₂(µ-L)ₙLₘ]ₚ wherein R¹ and R⁴ are independently selected alkyl, alkoxyalkyl, fluoroalkyl: having from 1 to 10 carbon atoms, cycloaliphatic, and aryl: having from 4 to 12 carbon atoms; R² and R³ are independently selected from hydrogen, alkyl, alkoxyalkyl, fluoroalkyl, alkoxy: having from 1 to 10 carbon atoms, cycloaliphatic, and aryl: having from 4 to 12 carbon atoms; L is independently selected from the organic amide class of the form RCONR'R" wherein R and R' are independently linear or branched alkyl having from 1-10 carbon atoms and they can be connected to form a cyclic group (CH₂)_{q}, wherein q is from 4-6, preferably 5, and R" is selected from alkyl having from 1 to 4 carbon atoms and cycloalkyl having from 4 to 8 carbon atoms; n is a number selected from between 1 and 10, preferably 3, 4; m is selected from a number between 0 to 4; and p is selected from 1, 2; µ-L indicates L is connected to two metals, M, via µL's oxygen atom when p=2.
(iii) Group 2 amidinates with organic amides as adducts with a formula of [M(R¹NC(R²)NR³)₂(µ-L)ₙLₘ]ₚ wherein R¹ and R³ are independently selected from linear or branched alkyl, alkoxyalkyl, fluoroalkyl: from 1 to 10 carbon atoms, cycloaliphatic, and aryl: having from 4 to 12 carbon atoms; R² is independently selected from hydrogen, linear or branched alkyl, fluoroalkyl, alkoxy: having from 1 to 10 carbon atoms, cycloaliphatic, and aryl: having from 4 to 12 carbon atoms; L either bridging between two metal atoms or coordinating to one metal atom and being independently selected from the organic amide class RCONR'R", wherein R and R' are independently linear or branched alkyl having from 1-10 carbon atoms and they can be connected to form a cyclic group (CH₂)_{q}, wherein q is from 4-6, and R" is selected from alkyl having from 1 to 4 carbon atoms and cycloalkyl having from 4 to 8 carbon atoms; n is a number selected from between 0 and 4; m is selected from a number between 1 to 4; and p is selected from 1, 2; and µ-L indicates L is connected to two metals, M, via µL's oxygen atom when p=2.

This invention is related to Group 2 metal-containing complexes having both beta-ketonate or beta-ketoiminate or amidinate and organic amides and their solutions, which are useful for fabricating conformal metal containing films on substrates such as silicon, metal nitride, metal oxide and other metal layers via deposition processes, e.g., chemical vapor deposition (CVD), plasma enhanced chemical vapor deposition (PECVD), low pressure chemical vapor deposition (LPCVD), plasma enhanced atomic layer deposition (PE ALD) and atomic layer deposition (ALD). Such conformal metal containing films have applications ranging from computer chips, optical device, magnetic information storage, to metallic catalyst coated on a supporting material. In contrast to prior polydentate beta-ketoiminate precursors, the polydentate beta-ketoiminate ligands incorporate at least one amino organo imino functionality, which is in contrast to the literatures reported alkoxy group as the donating ligand.

One type of structure in the metal precursor is illustrated in structure A below having the formula: 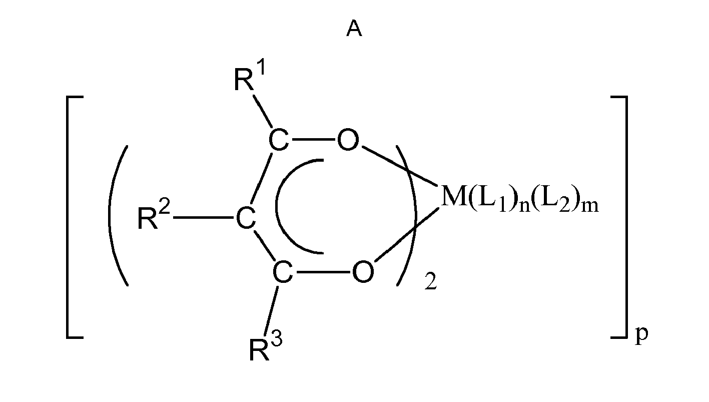 wherein M is selected from Mg, Ca, Sr, and Ba; R¹ and R³ are independently selected from linear or branched alkyl, alkoxyalkyl, fluoroalkyl: having from 1 to 10 carbon atoms, cycloaliphatic, and aryl having from 4 to 12 carbon atoms (i.e. where R¹ and/or R³ are alkyl, alkoxyalkyl, or fluoroalkyl they may have from 1 to 10 carbon atoms, and where R¹ and/or R³ are cycloaliphatic or aryl they may have from 4 to 12 carbon atoms); R² is independently selected from hydrogen, linear or branched alkyl, fluoroalkyl, alkoxy: having from 1 to 10 carbon atoms, cycloaliphatic, and aryl: having from 4 to 12 carbon atoms (i.e. where R² is alkyl, alkoxyalkyl, or fluoroalkyl it may have from 1 to 10 carbon atoms, and where R² is cycloaliphatic or aryl it may have from 4 to 12 carbon atoms); L₁ is independently selected from the organic amide class RCONR'R" wherein R and R' are linear or branched alkyl having from 1-10 carbon atoms and they can be connected to form a cyclic group (CH₂)_{q}, wherein q is from 4-6, and R" is selected from alkyl having from 1 to 4 carbon atoms and cycloalkyl having from 4 to 8 carbon atoms; L₂ is independently selected from H₂O, ROH wherein R is linear or branched alkyl having from 1-10 carbon atoms or a cyclic group (CH₂)_{q} wherein q is from 4-6, and an organic amide of the class RCONR'R" wherein R and R' are independently linear or branched alkyl having from 1-10 carbon atoms and they can be connected to form a cyclic group (CH₂)_{q}, wherein q is from 4-6 and neutral oxygen, and R" is selected from alkyl having from 1 to 4 carbon atoms and cycloalkyl having from 4 to 8 carbon atoms; n is a number selected from between 1 and 4; m is selected from a number between 0 to 4; and p is selected from 1, 2.

Another type of structure in the metal precursor is illustrated in structure B below having the formula: 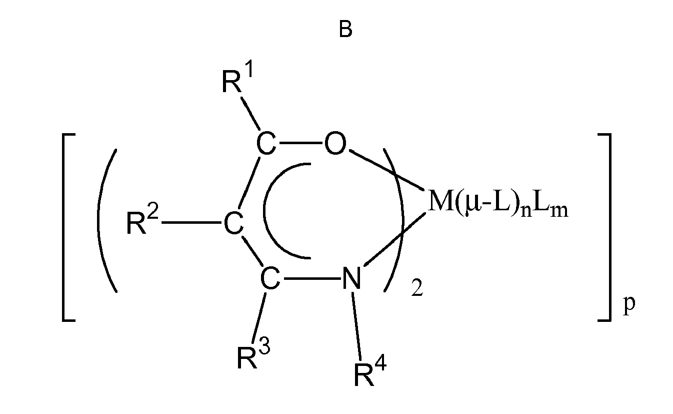 wherein M is selected from Mg, Ca, Sr, and Ba; R¹ and R⁴ are independently selected from linear or branched alkyl, alkoxyalkyl, fluoroalkyl: having from 1 to 10 carbon atoms, cycloaliphatic, and aryl: having from 4 to 12 carbon atoms (i.e. where R¹ and/or R⁴ are alkyl, alkoxyalkyl, or fluoroalkyl they may have from 1 to 10 carbon atoms, and where they are cycloaliphatic or aryl they may have from 4 to 12 carbon atoms); R² and R³ are independently selected from hydrogen, linear or branched alkyl, alkoxyalkyl, fluoroalkyl, alkoxy: having from 1 to 10 carbon atoms, cycloaliphatic, and aryl: having from 4 to 12 carbon atoms (i.e. where R² and/or R³ are alkyl, alkoxyalkyl, or fluoroalkyl they may have from 1 to 10 carbon atoms, and where they are cycloaliphatic or aryl they may have from 4 to 12 carbon atoms); L is independently selected from the organic amide class RCONR'R" wherein R and R' are independently linear or branched alkyl having from 1-10 carbon atoms and they can be connected to form a cyclic group (CH₂)_{q}, wherein q is from 4-6, and R" is selected from alkyl having from 1 to 4 carbon atoms and cycloalkyl having from 4 to 8 carbon atoms; n is a number selected from between 0 and 4 and m is selected from a number between 1 to 4, or n is a number selected from between 1 and 4 and m is selected from a number between 0 to 4; p is selected from 1, 2; and µ-L indicates L is connected to two metals, M, via µL's oxygen atom when p=2.

The third type of structure in the metal precursor is illustrated in structure C below having the formula: 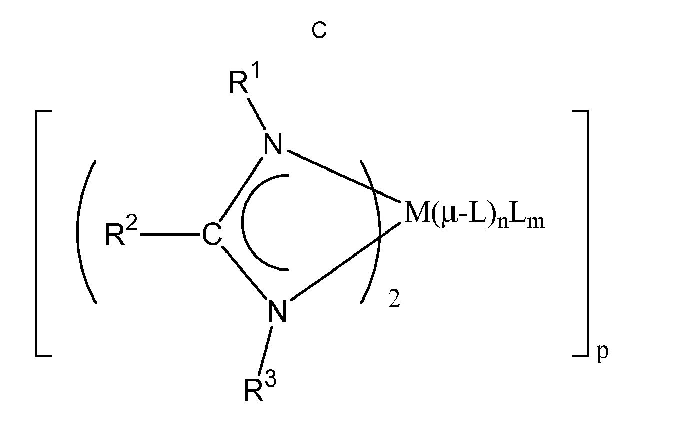 wherein M is selected from Mg, Ca, Sr, and Ba; R¹ and R³ are independently selected from linear or branched alkyl, alkoxyalkyl, fluoroalkyl: from 1 to 10 carbon atoms, cycloaliphatic, and aryl: having from 4 to 12 carbon atoms (i.e. where R¹ and/or R³ are alkyl, alkoxyalkyl, or fluoroalkyl they may have from 1 to 10 carbon atoms, and where they are cycloaliphatic or aryl they may have from 4 to 12 carbon atoms); R² is independently selected from hydrogen, linear or branched alkyl, fluoroalkyl, alkoxy: having from 1 to 10 carbon atoms, cycloaliphatic, and aryl: having from 4 to 12 carbon atoms (i.e. where R² is alkyl, alkoxyalkyl, or fluoroalkyl it may have from 1 to 10 carbon atoms, and where it is cycloaliphatic or aryl it may have from 4 to 12 carbon atoms); L is independently selected from the organic amide class RCONR'R" wherein R and R' are independently linear or branched alkyl having from 1-10 carbon atoms and they can be connected to form a cyclic group (CH₂)_{q}, wherein q is from 4-6, and R" is selected from alkyl having from 1 to 4 carbon atoms and cycloalkyl having from 4 to 8 carbon atoms; n is a number selected from between 0 and 4 and m is selected from a number between 1 to 4, or n is a number selected from between 1 and 4 and m is selected from a number between 0 to 4; p is selected from 1, 2; and µ-L indicates L is connected to two metals, M, via µL's oxygen atom when p=2.

These metal-containing complexes having both beta-ketonate or beta-ketoiminate ligands and organic amides can be employed as potential precursors to make thin metal or metal oxide films via either the chemical vapor deposition (CVD) or atomic layer deposition (ALD) method at temperatures less than 500 °C. The CVD process can be carried out with or without reducing or oxidizing agents, whereas an ALD process usually involves the employment of another reactant, such as a reducing agent or oxidizing agent.

For multi-component metal oxides such as STO and BST, these metal-containing complexes, having beta-ketonate or beta-ketoiminate or amidinate ligands and organic amides, can be delivered in vapor phase into a CVD or ALD reactor via well-known bubbling or vapor draw techniques as strontium or barium sources. The titanium source is selected from titanium alkoxides or beta-diketonates such as Ti(OPrⁱ)₄, Ti(tmhd)₂(OPrⁱ)₂, where Prⁱ = isopropyl, where tmhd = 2,2,6,6-tetramethyl-3,5-heptanedionate, Ti(mpd)(tmhd)₂, where mpd = 2-methyl-2,4-pentanedioxy, Ti(4-(2-methylethoxy)imino-2-pentanoate)₂, and analogous titanium ligands and derivatives. A direct liquid delivery method can also be employed by dissolving the titanium, strontium as well as barium complexes in a suitable solvent or a solvent mixture to prepare a solution with a molar concentration from 0.001 to 2 M, depending the solvent or mixed- solvents employed. Oxidizing agents for the deposition process include oxygen, water, hydrogen peroxide, oxygen plasma, nitrous oxide, and ozone. In a preferred embodiment, the multi-component metal oxide films are grown in the temperature range of 200 to 500°C, preferably 250 to 350°C whereby an amorphous STO or BST films are obtained. A thermal annealing is needed to convert the resulting films from amorphous into crystalline form. The annealing can be conducted at a higher temperature 500 to 1200°C under oxidizing conditions, preferably in the range of 500 to 700°C for high k dielectrics in DRAM applications. The thickness of the STO or BST film is in the range of 1 nm to 500nm, preferably 2nm to 10nm, deposited on compatible substrates including platinum(Pt), RuO₂, SrRuO₃, silica, silicon nitride, and silicon. The process chamber pressure may preferably be from about 0.1 Torr (13 Pa) to 100 Torr (13 kPa), and more preferably from about 0.1 Torr (13 Pa) to 5 Torr (670 Pa).

The solvent employed in solubilizing the precursor for use in a deposition process may comprise any compatible solvent or their mixture, including: aliphatic hydrocarbons, aromatic hydrocarbons, ethers, esters, nitrites, and alcohols. The solvent component of the solution preferably comprises a solvent selected from: glyme solvents having from 1 to 20 ethoxy -(C₂H₄O)- repeat units; C₂-C₁₂ alkanols, organic ethers selected from dialkyl ethers comprising C₁-C₆ alkyl moieties, C₄-C₈ cyclic ethers; C₁₂-C₆₀ crown O₄-O₂₀ ethers (wherein C is the number of carbon atoms in the ether compound and O is the number of oxygen atoms in the ether compound); C₆-C₁₂ aliphatic hydrocarbons; C₆-C₁₈ aromatic hydrocarbons; organic esters; organic amines, polyamines and organic amides.

Another class of solvents that offers advantages is the organic amide class of the form RCONR'R", wherein R and R' are independently linear or branched alkyl having from 1-10 carbon atoms, and they can be connected to form a cyclic group (CH₂)_{q}, wherein q is from 4-6, preferably 5, and R" is selected from alkyl having from 1 to 4 carbon atoms and cycloalkyl having 4 to 8 carbon atoms. N-methyl pyrrolidinone (NMP), *N,N-*Diethylformamide (DEF), *N,N*-Diethylacetamide (DEAC), *N,N-*Dimethylacetamide (DMAC),and N-cyclohexyl 2-pyrrolidinone are examples.

The following examples illustrate the preparation of the metal-containing complexes with beta-diketone or beta-ketoiminate ligands as well as their use as precursors in metal-containing film deposition processes.

### Example 1

### Synthesis of Ba₂(hfac)₄(NMP)₅

0.52g (3.80 mmol) of BaH₂ was loaded in flask with 15mL of toluene. To this flask was added 2.15g (21.70 mmol) NMP followed by addition of 1.57g (7.60 mmol) 1,1,1,5,5,5-hexafluoro-2,4-pentanedione ("hfac"). Stirred reaction mixture at room temperature until bubbling ceased after 5-10 minutes. Removed volatiles under vacuum and noticed the formation of a white solid. Upon completion of evaporation, a liquid was left which was taken up in hexane and recrystallized in -40°C freezer. Decanted out hexanes from recrystallized solid and solid taken up in toluene and recrystallized again in freezer. Decanted toluene from crystals. Crystals identified by crystal structure analysis as the dimer Ba₂(hfac)₄(NMP)₅.

### Example 2

### Synthesis of Sr₂(tmhd)₄(NMP)₄

0.50g (5.58 mmol) of SrH₂ was loaded into a 100mL Schlenk flask with 5g of NMP. To this flask was added 2.06g (11.16 mmol) 2,2,6,6-tetramethyl-3,5-heptanedione ("tmhd") in 5g of NMP and bubbling occurred. Left to stir over night at room temperature. Reaction mixture was subjected to vacuum transferred at 65-70°C leaving behind a grey solid weighing 2.86g. Recrystallized in a 1:1 solution of hexane to pentane in -40°C freezer to afford clear needle like crystals that were identified by crystal structure analysis as the dimer Sr₂(tmhd)₄(NMP)₄.

### Example 3

### Synthesis of Sr₂(tmhd)₄(DEAC)₃

To a suspension of 0.50g (5.58 mmol) SrH₂ in hexanes was added 2.10g (11.16 mmol) 2,2,6,6-tetramethyl-3,5-heptanedione ("tmhd") and 2.60g (22.32 mmol) N,N-diethylacetamide (DEAC) in hexanes at room temperature. Bubbling and heat were given off and the suspension turned to solution after approximately 10 minutes.. The reactin mixture was stirred for 16 hours and removal of hexanes to afford an oil that was set up for vacuum transfer and heated at 80°C under 300mTorr (40 Pa) for several hours. 2.38g of clear residual slurry (68% yield) was collected and recrystallization in pentane an -40°C gave rise to clear crystals. The crystals were identified by crystal structure analysis as Sr₂(tmhd)₄(DEAC)₃.

### Example 4

### Synthesis of Ba₂(tmhd)₄(NMP)₄(H₂O)₂

0.50g (3.59 mmol) of BaH2 was loaded into a 100mL Schlenk flask with 15mL of hexane. To this flask was added a solution of 1.32g (7.18 mmol) tmhd and 1.42 (14.35 mmol) NMP in 5mL hexane and bubbling was witnessed. After approximately 6 hours, the hexane was evaporated under vacuum leaving behind a grey solid weighing 2.32g. Recrystallized in octane giving clear hexagonal-like crystals that were sent for crystal structure analysis and confirmed as the proposed dimer. A 90% yield was obtained based off of crude.

### Example 5

### Synthesis of 2,2-dimethyl-5-(iso-propylamino)-3-hexanone

To a solution of 15.00g (105.49mmol) 2,2-dimethyl-3,5-hexanedione in 50mL of toluene loaded with 30.00g sodium sulfate was added 12.47g (210.97mmol) isopropylamine. The mixture was refluxed for 4 days. Removal of toluene resulted in a yellow oil, which was subjected to vacuum transfer at 80°C under 125mTorr (17 Pa). 16.5 g of clear oil was obtained with a yield of 84%. ¹H NMR (500 MHz, C₆D₆): δ= 11.50 (s, 1 H), 5.16 (s, 1 H), 3.11 (m, 1 H), 1.49 (s, 3H), 1.31 (s, 9H), 0.79 (d, 6H).

### Example 6

### Synthesis of 2,2-dimethyl-5-(sec-butylamino)-3-hexanone

To a solution of 5.00g (35.16mmol) 2,2-dimethyl-3,5-hexanedione in 20mL of toluene loaded with 10.00g of sodium sulfate was added 5.14g (70.32mmol) sec-butylamine. The mixture was refluxed 3 days. 4.90g of light yellow oil was obtained after work-up. The isolated yield was 71%. ¹H NMR (500 MHz, C₆D₆): δ= 11.52 (s, 1H), 5.18 (s, 1 H), 2.95 (m, 1 H), 1.51 (s, 3H), 1.31 (s, 9H), 1.14 (s, 2H), 0.80 (d, 3H), 0.67 (t, 3H).

### Example 7

### Synthesis of bis(2,2-dimethyl-5-(iso-propylamino)-3-hexanonato)barium NMP adduct

To a suspension of 1g (1.66mmol) Ba(N(SiMe₃)₂)₂(THF)₂ in hexane was added 0.61g (3.32mmol) of 2,2-dimethyl-5-(iso-propylamino)-3-hexanone and 0.66g (6.64mmol) of NMP in hexane at room temperature. The reaction mixture turned to solution after approximately 30 minutes. After stirring for 16 hours, hexane was evaporated under vacuum to provide 0.95g of a white solid. The white solid was heated in hexane, filtered, and recrystallized an -40°C to give rise to foggy-white crystals. ¹H NMR (500 MHz, C₆D₆): δ= 5.06 (s, 1H), 3.75 (m, 1 H), 2.42 (s, 3H), 2.40 (t, 2H), 1.98 (t, 2H), 1.83 (s, 3H), 1.44 (s, 9H), 1.34 (d, 6H), 1.17 (m, 2H).

### Example 8

### Synthesis of bis(2,2-dimethyl-5-(sec-butylamino)-3-hexanonato)strontium NMP adduct

To a solution of 1 g (1.81 mmol) of Sr(N(SiMe₃)₂)₂(THF)₂ in hexanes was added 0.66g (3.62mmol) of 2,2-dimethyl-5-(sec-butylamino)-3-hexanone and 0.72g (7.24mmol) of NMP in hexanes at room temperature. After stirring for 16 hours, hexanes were evaporated under vacuum. Recrystallization in hexanes at -20°C resulted in a opaque white solid. ¹H NMR (500 MHz, C₆D₆): δ= 5.06 (s, 1 H), 3.75 (m, 1 H), 2.46 (s, 3H), 2.42 (t, 2H), 2.07 (t, 2H), 1.90 (s, 3H), 1.47 (s, 9H), 1.47 (d, 6H), 1.21 (m, 2H).

### Example 9

### Synthesis of bis(2,2-dimethyl-5-(sec-butylamino)-3-hexanonato)strontium DEAC adduct

To a solution of 1g (1.81mmol) Sr(N(SiMe₃)₂)₂(THF)₂ in 10mL hexanes at room temperature was added 0.71 g (3.62mmol) of 2,2-dimethyl-5-(sec-butylamino)-3-hexanone and 0.83g (7.24mmol) DEAC in 10mL hexanes. Reaction was stirring for 16 hours. Evaporation of hexanes afforded a wet off-white solid. ¹H NMR (500 MHz, C₆D₆): δ= 5.05 (s, 1 H), 3.59 (m, 1 H), 3.17 (q, 2H), 2.51 (q, 2H), 1.96 (s, 3H), 1.79 (s, 3H), 1.55 (s, 9H) 1.44 (m, 2H), 1.44 (d, 3H), 1.03 (t, 3H) 0.96 (t, 3H), 0.56 (t, 3H).

## Claims

1. A metal containing complex represented by a structure selected from: 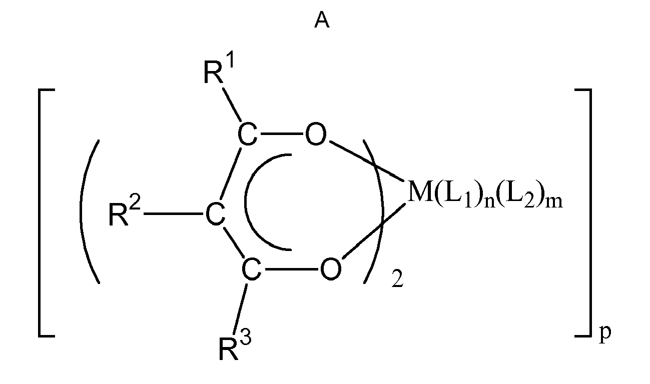 wherein M is selected from Mg, Ca, Sr, and Ba; R¹ and R³ are independently selected from linear or branched alkyl, alkoxyalkyl, fluoroalkyl: having from 1 to 10 carbon atoms, cycloaliphatic, and aryl having from 4 to 12 carbon atoms; R² is independently selected from hydrogen, linear or branched alkyl, fluoroalkyl, alkoxy: having from 1 to 10 carbon atoms, cycloaliphatic, and aryl: having from 4 to 12 carbon atoms; L₁ is independently selected from the organic amide class RCONR'R" wherein R and R' are independently linear or branched alkyl having from 1-10 carbon atoms and they can be connected to form a cyclic group (CH₂)_{q}, wherein q is from 4-6, and R" is selected from alkyl having from 1 to 4 carbon atoms and cycloalkyl having from 4 to 8 carbon atoms; L₂ is independently selected from H₂O, ROH wherein R is linear or branched alkyl having from 1-10 carbon atoms or a cyclic group (CH₂)_{q} wherein q is from 4-6, and an organic amide of the class RCONR'R" wherein R and R' are independently linear or branched alkyl having from 1-10 carbon atoms and they can be connected to form a cyclic group (CH₂)_{q}, wherein q is from 4-6, and R" is selected from alkyl having from 1 to 4 carbon atoms and cycloalkyl having from 4 to 8 carbon atoms; n is a number selected from between 1 and 4; m is selected from a number between 0 to 4; and p is selected from 1, 2; 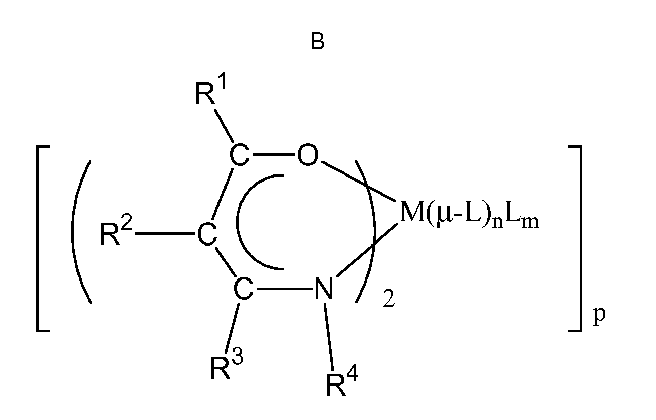 wherein M is selected from Mg, Ca, Sr, and Ba; R¹ and R⁴ are independently selected from linear or branched alkyl, alkoxyalkyl, fluoroalkyl: having from 1 to 10 carbon atoms, cycloaliphatic, and aryl: having from 4 to 12 carbon atoms; R² and R³ are independently selected from hydrogen, linear or branched alkyl, alkoxyalkyl, fluoroalkyl, alkoxy: having from 1 to 10 carbon atoms, cycloaliphatic, and aryl: having from 4 to 12 carbon atoms; L is independently selected from the organic amide class RCONR'R" wherein R and R' are independently linear or branched alkyl having from 1-10 carbon atoms and they can be connected to form a cyclic group (CH₂)_{q}, wherein q is from 4-6, and R" is selected from alkyl having from 1 to 4 carbon atoms and cycloalkyl having from 4 to 8 carbon atoms; n is a number selected from between 0 and 4; m is selected from a number between 1 to 4; p is selected from 1, 2; and µ-L indicates L is connected to two metals, M, via µL's oxygen atom when p=2; and, 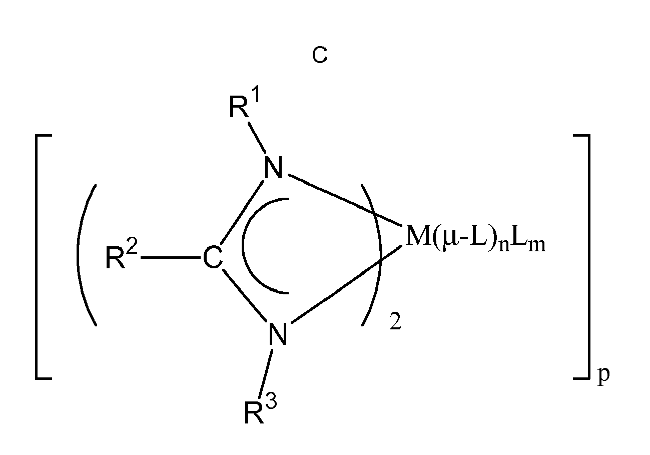 wherein M is selected from Mg, Ca, Sr, and Ba; R¹ and R³ are independently selected from linear or branched alkyl, alkoxyalkyl, fluoroalkyl: from 1 to 10 carbon atoms, cycloaliphatic, and aryl: having from 4 to 12 carbon atoms; R² is independently selected from hydrogen, linear or branched alkyl, fluoroalkyl, alkoxy: having from 1 to 10 carbon atoms, cycloaliphatic, and aryl: having from 4 to 12 carbon atoms; L is independently selected from the organic amide class RCONR'R" wherein R and R' are independently linear or branched alkyl having from 1-10 carbon atoms and they can be connected to form a cyclic group (CH₂)_{q}, wherein q is from 4-6, and R" is selected from alkyl having from 1 to 4 carbon atoms and cycloalkyl having from 4 to 8 carbon atoms; n is a number selected from between 0 and 4; m is selected from a number between 1 to 4; p is selected from 1, 2; and µ-L indicates L is connected to two metals, M via µL's oxygen atom when p=2.

2. The metal containing complex of Claim 1 wherein M is selected from calcium, strontium, barium and mixtures thereof.

3. The metal containing complex of Claim 1 or 2, wherein the metal containing complex is of structure A, and L₁ and L₂ are selected from N-methyl pyrrolidinone (NMP), *N,N*-Diethylformamide (DEF), *N,N*-Diethylacetamide (DEAC), *N,N-*Dimethylacetamide (DMAC), N-cyclohexyl 2-pyrrolidinone, H₂O, and ROH.

4. The metal containing complex of Claim 1 or 2, wherein the metal containing complex is of structure A; R¹ and R³ are selected from t-butyl and t-pentyl; and R² is selected from hydrogen, methyl and ethyl.

5. The metal containing complex of Claim 1 structure A wherein M is strontium, R¹ and R³ are each t-butyl, R² is hydrogen, L₁ and L₂ are each N-methyl-pyrrolidone, n=1.5, m=0.5, and p=2.

6. The metal containing complex of Claim 1 structure A wherein M is strontium, R¹ and R³ are each t-butyl, R² is hydrogen, L₁ is *N,N-*Diethylacetamide (DEAC), n =1.5, m=0, and p=2.

7. The metal containing complex of Claim 1 structure A wherein M is barium, R¹ and R³ are each CF₃, R² is hydrogen, L₁ and L₂ are each N-methyl-pyrrolidone, n =1.5, m=1, and p=2.

8. The metal containing complex of Claim 1 structure A wherein M is barium, R¹ and R³ are each t-butyl, R² is hydrogen, L₁ is N N-methyl-pyrrolidone, n=2, L₂=H₂O, m=1, and p=2.

9. The metal containing complex of Claim 1 or 2, wherein the metal containing complex is of structure B; R¹ and R³ are independently selected from t-butyl and t-pentyl; R² is selected from hydrogen, methyl and ethyl; and R⁴ is selected from iso-propyl, t-butyl, sec-butyl, t-pentyl and mixtures thereof.

10. The metal containing complex of Claim 1 or 2, wherein the metal containing complex is of structure C; R¹ and R³ are independently selected from t-butyl and t-pentyl; and R² is selected from hydrogen, methyl and ethyl.

11. The metal containing complex of Claim 9 or 10, wherein L is selected from N-methyl pyrrolidinone ("NMP"), *N,N*-Diethylformamide (DEF), *N,N*-Diethylacetamide (DEAC), *N,N*-Dimethylacetamide (DMAC),and N-cyclohexyl 2-pyrrolidinone.

12. The metal containing complex of any preceding claim dissolved in a solvent selected from glyme solvents having from 1 to 20 ethoxy -(C₂H₄O)- repeat units; C₂-C₁₂ alkanols, organic ethers; C₆-C₁₂ aliphatic hydrocarbons; C₆-C₁₈ aromatic hydrocarbons; organic esters; organic amines; and polyamines and organic amides.

13. A vapor deposition process for forming a conformal multi-component metal oxide thin film on a substrate wherein at least two metal containing complexes and an oxygen containing agent are introduced to a deposition chamber and a multi-component metal oxide film is deposited on a substrate, which process comprises using at least two metal containing complexes according to any one of Claims 1 to 12, each having different metals.

14. The process of Claim 13 wherein the oxygen containing agent is selected from N₂O, O₂, H₂O₂, H₂O, ozone, O₂ plasma, organic peroxide, and mixtures thereof.

15. The process of Claim 13 or 14 wherein a third metal containing complex is selected from titanium containing alkoxides and beta-ketonates and mixtures thereof.
